(19) Europäisches Patentamt
European Patent Office
Office européen des brevets

(11) **EP 2 570 114 A1**

(12) **EUROPEAN PATENT APPLICATION**
published in accordance with Art. 153(4) EPC

(43) Date of publication:
**20.03.2013 Bulletin 2013/12**

(51) Int Cl.:
***A61K 8/97*** (2006.01)    ***A61Q 17/04*** (2006.01)
***A61Q 19/00*** (2006.01)

(21) Application number: **10851458.9**

(22) Date of filing: **16.09.2010**

(86) International application number:
**PCT/KR2010/006338**

(87) International publication number:
**WO 2011/142507 (17.11.2011 Gazette 2011/46)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **12.05.2010 KR 20100044593**

(71) Applicant: **Athena Co., Ltd.
Suwon, Gyeonggi-do 441-100 (KR)**

(72) Inventors:
• **PARK, En-Kyung
Yongin-si
Gyeonggi-do 448-140 (KR)**
• **PARK, Sang-Hee
Seoul 121-080 (KR)**

• **Jung, Won-Soon
Suwon-si
Gyeonggi-do 442-081 (KR)**
• **LEE, Mi-Jin
Seoul 152-090 (KR)**
• **JO, Young Hyun
Gwangju 502-776 (KR)**
• **KANG, Sung-Pil
Mokpo-si
Jeollanam-do 530-370 (KR)**
• **KIM, Hae Seop
Mokpo-si
Jeollanam-do 530-350 (KR)**

(74) Representative: **Winkler, Andreas Fritz Ernst
Boehmert & Boehmert
Pettenkoferstrasse 20-22
80336 München (DE)**

(54) **COSMETIC COMPOSITION CONTAINING GRACILARIOPSIS CHORDA EXTRACT FOR BLOCKING UV RAYS**

(57) The present invention relates to a cosmetic composition for blocking ultraviolet (UV) rays comprising *Gracilariopsis chorda* extract. The cosmetic composition of this invention for UV protection has excellent activities for inhibition of direct UV illumination on skin by absorbing UV-A, and not only prevents oxidation of cell membrane but also recovers skin damages caused by UV in a significant manner. In addition, the cosmetic compositions of the present invention have the advantage of not only safety on skin but also stability as cosmetics, since the cosmetics of this invention includes *Gracilariopsis chorda* extract derived from natural-occurring materials as an active ingredient.

**Fig. 2**

Red: disodium phenyldibenzyimidazole tetrasulfonate 100 ppm
Black: *Gracilariopsis chorda* extracts 100 ppm
Green: *Gracilariopsis chorda* extracts 200 ppm
Blue: *Gracilariopsis chorda* extracts 300 ppm

**Description**

**CROSS-REFERENCE TO RELATED APPLICATIONS**

This application claims priority from Korean Patent Application No. 2010-0044593, filed on 12 May 2010, in the Korean Intellectual Property Office, the disclosure of which is incorporated herein by reference.

**BACKGROUND OF THE INVENTION**

**FIELD OF THE INVENTION**

The present invention relates to a *Gracilariopsis chorda* extract having UV-A-blocking activities and a cosmetic composition using the same.

**DESCRIPTION OF THE RELATED ART**

UV (ultraviolet) rays are a light having a wavelength of less than 400 nm emitted from sun, and divided into long-wave UV (UV-A; 320-400 nm), medium-wave UV (UV-B; 280-320 nm), short-wave UV (UV-C; 200-280 nm). UV rays induce photobiological responses on skin to cause diverse diseases including sun burn, pigmentation, photoaging or skin cancer, and to develop various skin damages depending on a wavelength.
UV-C causes sterilization and cell death whereas it is difficult to reach skin because most UV-C is absorbed in an ozone layer. UV-B arrives on epidermal basal layer and upper dermis of skin to persist skin scaling and to induce sun burn such as erythema and blister, pigmentation, cell membrane damage and skin cancer. Interestingly, UV-A reach dermis to induce immediate pigmentation and delayed erythema, phototoxicity, photoallergic responses, chronic photoaging caused by free radical production, and to reduce skin elasticity by denaturing elastic fiber and fiber in dermis, resulting in wrinkle or skin atrophy. Thus, the use of a UV-blocking agent is essential to protect skin from UV.
Conventional UV-blocking agents are widely classified into a UV-absorbing agent as a chemical blocker and a UV-scattering agent as a physical blocker. The UV-absorbing agent absorbs UV rays to inhibit direct UV illumination on skin, and includes a synthetic absorber such as benzophenone, methyl anthranilate, paraaminobenzoic acid derivatives, paramethoxy cinnamate derivatives or salicylic acid derivatives. However, the synthetic absorber exhibits serious demerits such as skin sttimulus and skin toxicity. Meanwhile, the UV-scattering agent scatters or reflects UV rays on skin to block UV penetration into skin, and has been utilized as a powder form including titanium dioxide, zinc oxide, mica, talc, kaolin, and so forth. The UV-scattering agent has the following drawbacks: (a) induction of skin stimuli due to residual agent on skin; (b) whitening effect that remains white traces on skin in application because of hardship in dispersion and solubility of agent type. In addition, skin application of the UV-scattering agent gives an unpleasant feeling. There have been urgently demanded these natural UV-blocking agents with moderate skin stimuli and few shortcomings since they are most chemical synthetic materials with strong skin stimuli and diverse drawbacks.
UV-blocking substances derived from natural-occurring materials are mostly UV-absorbing substances that show mild stimuli and exert pharmacological functions such as an anti-inflammatory, astringent or anti-bacterial activity, and thus many studies have been attempted. For example, Korean Publication Patent No. 10-1995-0012443 disclosed a cosmetic composition containing herb extract with UV-absorbing activity, and Korean Publication Patent No. 10-2006-0088347 disclosed a cosmetic composition for absorbing UV comprising green tea and fennel extract. However, since conventional natural UV-blocking agents are a few and their effects are superficial, few examples applicable in cosmetics have been reported.
In the mean time, *Gracilariopsis chorda* belongs to seaweeds of family *Gracilariaceae* distributed in Korea, China and Japan, particularly found in sublittoral zone around southwest coast of Korea. As a study for *Gracilariaceae* spp., Korean Publication Application Patent No. 10-2006-0050782 described noodles containing *Gracilariaceae* and preparation methods thereof, and also disclosed compositions for preventing inflammatory diseases comprising as an active ingredient a chemical compound derived from *Gracilariaceae* extract in view of physiological activities of *Gracilariaceae.* In addition, although a variety of reports have been published to prepare numerous foods, agar-agars and pulps, few studies for cosmetics using *Gracilariaceae* have been reported. Moreover, effects of *Gracilariaceae* for UV protection have been remained to be elucidated.
Throughout this application, various patents and publications are referenced, and citations are provided in parentheses. The disclosure of these patents and publications in their entities are hereby incorporated by references into this application in order to more fully describe this invention and the state of the art to which this invention pertains.

## SUMMARY OF THE INVENTION

The present inventors have made intensive researches to develop a cosmetic composition containing a natural-occurring UV-absorbing component. As a result, we have discovered that *Gracilariopsis chorda* extract has excellent effects on UV-A-blocking, anti-oxidation and cell regeneration, and exhibits not only safety on skin but also stability as cosmetics.

Accordingly, it is an object of this invention to provide a cosmetic composition for blocking ultraviolet (UV) rays, comprising *Gracilariopsis chorda* extract.

Other objects and advantages of the present invention will become apparent from the detailed description to follow taken in conjugation with the appended claims and drawings.

## BRIEF DESCRIPTION OF THE DRAWINGS

Fig. 1 is a graph showing anti-oxidative effects of *Gracilariopsis chorda* extract of the present invention,

Fig. 2 shows a spectrum measuring UV absorbing capability of *Gracilariopsis chorda* extract of the present invention.

Fig. 3 is a spectrum measuring UV transmission of *Gracilariopsis chorda* extract of the present invention.

Fig. 4 represents a spectrum comparing UV absorbing capability of *Gracilariopsis chorda* extract of the present invention with *Porphyra tenera* extract and *Gelidium Cartilagineum* extract prepared in Comparative Preparation Examples 1 and 2, respectively.

## DETAILED DESCRIPTION OF THIS INVETNION

The present inventors have made intensive researches to develop a cosmetic composition containing a natural-occurring UV-absorbing component. As a result, we have discovered that *Gracilariopsis chorda* extract has excellent effects on UV-A-blocking, anti-oxidation and cell regeneration, and exhibits not only safety on skin but also stability as cosmetics.

*Gracilariopsis chorda* belongs to seaweeds of family *Gracilariaceae* distributed in Korea, China and Japan, particularly found in sublittoral zone around southwest coast of Korea.

The method to prepare *Gracilariopsis chorda* extract may be carried out according to conventional methods well-known to those ordinarily skilled in the art. Preferably, an extraction solvent is a conventional solvent useful in the art and may utilize at least one selected from the group consisting of purified water, methanol, ethanol, glycerin, ethylacetate, butylglycol, propyleneglycol, dichloromethane and hexane.

Since *Gracilariopsis chorda* extract has excellent effects on UV-A absorption, UV-A-blocking, anti-oxidation, cell regeneration, and safety on skin, it is sufficient to utilize it as a natural UV-blocking component in a cosmetic composition.

The cosmetic composition of the present invention includes *Gracilariopsis chorda* extract at a concentration of 1-20% by weight. The cosmetic composition has low effects where *Gracilariopsis chorda* extract at a concentration of less 1% by weight, and the cosmetic composition exhibits no significantly enhanced effects in more 20% by weight.

The cosmetic composition of the present invention may further include other UV-blocking agents, thereby contributing to enhancement of UV-blocking effects Additive UV-blocking agents may utilize any one known to those ordinarily skilled in the art, for example including an organic or inorganic UV-blocking component. Examples of organic UV-blocking component include, but is not limited to, at least one selected from the group consisting of octyl methoxy cinnamate, octyl salicylate, octocrylene, benzophenone-3, benzophenone-4, benzophenone-8, butyl methoxy dibenzoyl methane, oxy benzene, octyl triazone, menthyl anthranilate, 3,4-methylbenzylidencamphor, 2-phenylbenzimidazole-5-sulfonic acid, disodium phenyldibenzyimidazole tetrasulfonate, isoamyl-p-methoxylcinnamate and bis-ethylhexyloxyphenol methoxyphenyl triazine, and the non-limiting examples of inorganic UV-blocking component include at least one selected from the group consisting of titanium dioxide, zinc oxide and iron oxide.

In a certain embodiment, the cosmetic composition further includes laver *(porphyry tenera)* extract or *Gelidium Cartilagineum* extract.

In other certain embodiment, the cosmetic compositions of this invention may contain not only UV-blocking compounds as active ingredients, but also components utilized in the cosmetic compositions in the art, for example including conventional additives and carriers such as stabilizers, solubilizers, surfactants, vitamins, colorants and perfumes.

The cosmetic compositions of this invention for skin protection may be formulated in the art in a wide variety of forms, for example, including a solution, a suspension, an emulsion, a paste, a gel, a cream, a lotion, a powder a soap, a surfactant-containing cleanser, an oil, a powder foundation, an emulsion foundation, a wax foundation and a spray, but is not limited to. Specifically, the cosmetic compositions of this invention may be formulated in the form of skin softner, nutrient liquid, nutrient cream, massage cream, essence, eye cream, cleansing cream, cleansing foam, cleansing water, pack, spray or powder.

Where the cosmetic composition is in the form of paste, cream or gel, it may contain animal and vegetable fats, waxes, paraffins, starch, tragacanth, cellulose derivatives, polyethylene glycols, silicones, bentonites, silica, talc or zinc oxide, and so forth.

In the formulation of powder or spray, it may include lactose, talc, silica, aluminum hydroxide, calcium silicate or polyamide powder as a carrier, and spray may further comprise the customary propellants such as chlorofluorohydrocarbons, propane/butane or dimethyl ether.

The formulation of solution and emulsion may contain solvent, solubilizer and emulsifier, for example water, ethanol, isopropanol, ethyl carbonate, ethyl acetate, benzyl alcohol, benzyl benzoate, propylene glycol, 1,3-butylglycol, oils, glycerol fatty esters, polyethylene glycol or fatty acid esters of sorbitan.

The formulation of suspension may include liquid diluents, for example water, ethanol or propylene glycol, suspending agents such as ethoxylated isosteary alcohols, polyoxyethylene sorbitol esters and poly oxyethylene sorbitan esters, micocrystalline cellulose, aluminum metahydroxide, bentonite, agar or tragacanth.

The formulation of cleansing compositions with surfactant may comprise aliphatic alcohol sulfate, aliphatic alcohol ether sulfate, sulfosucinnate monoester, isothinate, imidazolium derivatives, methyltaurate, sarcocinate, fatty acid amide ether sulfate, alkyl amido betain, aliphatic alcohol, fatty acid glyceride, fatty acid diethanolamide, vegetable oil, lanoline derivatives or ethoxylated glycerol fatty acid ester.

The cosmetic compositions of this invention may be formulated according to the methods known to those ordinarily skilled in the art besides *Gracilariopsis chorda* extract as a UV-blocking component.

The features and advantages of this invention are will be summarized as follows:

(a) the present invention provides a cosmetic composition for blocking ultraviolet (UV) rays comprising *Gracilariopsis chorda* extract.
(b) the cosmetic composition of this invention for UV protection has excellent activities for inhibition of direct UV illumination on skin by absorbing UV-A, and not only prevents oxidation of cell membrane but also recovers skin damages caused by UV in a significant manner.
(c) In addition, the cosmetic compositions of the present invention have the advantage of not only safety on skin but also stability as cosmetics, since the cosmetics of this invention includes *Gracilariopsis chorda* extract derived from natural-occurring materials as an active ingredient,

The present invention will now be described in further detail by examples. It would be obvious to those skilled in the art that these examples are intended to be more concretely illustrative and the scope of the present invention as set forth in the appended claims is not limited to or by the examples.

**EXAMPLES**

**Effects of *Gracilariopsis chorda* extracts on Cell Growth, Anti-Oxidation and UV-A Absorption**

**1. Preparation of *Gracilariopsis chorda* extract (Preparation Example 1), and laver (*Porphyra tenera*) extract and *Gelidium Cartilagineum* extract (Comparative Preparation Examples 1-2)**

*Gracilariopsis chorda* used in Preparation Example 1 was harvested in the vicinity of Jindo, Jeollanam-do, and washed with flowing purified water. After heat-drying in a 55°C dry oven, the plant was pulverized and then filtered with 1 mm$^2$ mesh. *Gracilariopsis chorda* soaking solution was prepared by mixing the powder (50 g) with 70% ethanol (4,950 g), and extracted three times for 3 hrs under dark condition at 45°C. The extract was cooled by 20°C and filtered two times with 0.2 mm Watman paper (No. 5A Watman paper), followed by completely removing the solvent in the resulting liquid using a rotor concentrator Subsequently, the remnants were suspended in purified water (200 g) and fractionated by adding the same weight of hydrated butanol. As a result, the aqueous layer was concentrated and the residual butanol was removed, leading to prepare final *Gracilariopsis chorda* extract (50 g).

The laver used in Comparative Preparation Example 1 was dried domestic laver (*Porphyra tenera*) purchased from the agricultural and marine products wholesale center. *Porphyra tenera* extract was prepared by utilizing the dried laver (50 g) according to the same method as described in Preparation Example 1.

*Gelidium Cartilagineum* used in Comparative Preparation Example 2 was dried domestic agar-agar purchased from the agricultural and marine products wholesale center. *Gelidium Cartilagineum* extract was prepared by utilizing the dried agar-agar (50 g) according to the same method as described in Preparation Example 1.

2. Effect of *Gracilariopsis chorda* extract on cell growth

Effect of *Gracilariopsis chorda* extract prepared in Preparation Example 1 was tested on cell growth. In addition, the effect of disodium phenyldibenzyimidazole tetrasulfonate on cell growth as a UV-blocking agent generally utilized in the art was compared with that of *Gracilariopsis chorda* extract. Cell growth and toxicity test was carried out according to the method described in the document (Mosmann T, et al., Rapid Colorimetric Assay for Cellular Growth and Survival: Application to Proliferation and Cytotoxicity Assays, J. Immunol. Meth., 65: 55-63 (1983)). DMEM (Dulbeco's Modified Eagle's Medium) containing 10% FBS (fetal bovine serum) was utilized in cell growth and toxicity test in which human fibroblast cell line (KCLB, CCD-986) was inoculated on a 24-well plate with $1 \times 10^4$ cells/ml. At 24 hrs post-inoculation, the media was changed and *Gracilariopsis chorda* extract of Preparation Example 1 and synthetic UV blocking agent of Comparative Example was added depending on concentrations, followed by incubating for 3 days at 37°C in 5% $CO_2$ incubator. On the contrary, only the media were added in control. After MTT (Tetrazolium-based colorimetric) solution with 1 ml/well was added and incubated for 4 hrs at 37°C in 5% $CO_2$ incubator, the media was removed and DMSO (dimethylsulfoxide) was added with 200 μl per a well. The absorbance was measured at 550 nm, and cell growth rate was calculated according to the Formula below, as represented in Table 1.

$$\text{Effect on Cell Growth (\%)} = \frac{\text{Absorbance in Extract treatment} - \text{Absorbance in control}}{\text{Absorbance in control}} \times 100$$

**Table 1.**

| Extract concentration (%) | Cell growth rate (%) | |
|---|---|---|
| | *Gracilariopsis chorda* extract | disodium phenyldibenzyimidazole tetrasulfonate |
| 0 (control) | 100 | 100 |
| 0.01 | 100.21 | 100.15 |
| 0.03 | 100.59 | 100.10 |
| 0.05 | 105.67 | 99.51 |
| 0.1 | 107.43 | 98.60 |
| 0.15 | 110.32 | 97.54 |
| 0.3 | 114.13 | 95.21 |

In aforementioned Table 1, cell growth was enhanced in both *Gracilariopsis chorda* extract and synthetic UV blocking agent with a proviso that cell growth under the optimal condition is determined as 100%, As a result, disodium phenyldibenzyimidazole tetrasulfonate as a chemical synthetic UV blocking agent not only had no effect on cell growth but also showed low cellular toxicity at a concentration of not less than 0.05%. It could be appreciated that the present *Gracilariopsis chorda* extract has no cellular toxicity and exerts an effect on cell regeneration, in addition to enhancement on cell growth depending on increasing treatment concentrations of extract.

3. Effect of *Gracilariopsis chorda* extract on anti-oxidation

Effect of *Gracilariopsis chorda* extract prepared in Preparation Example 1 on anti-oxidation was determined by measuring electron donating ability (EDA) using DPPH (2,2-Diphenyl-1-picrylhydrazyl). EDA is a hallmark representing inhibitory activity of oxidation via donating an electron to free radicals. Where EDA is high, excellent anti-oxidative activity and superior scavenging ability for not only reactive oxygen species but also other radicals may be expected. *Gracilariopsis chorda* extract prepared in Preparation Example 1 was diluted as described in Table 2 below, and utilized in Examples 1-3. Free radical-scavenging ability of ascorbic acid (0.1%) solution as an anti-oxidant conventionally utilized in the art was compared with that of *Gracilariopsis chorda* extract.

**Table 2.**

| Test sample | Example 1 | Example 2 | Example 3 |
|---|---|---|---|
| Concentration (%) | 0.1% | 1.0% | 10% |

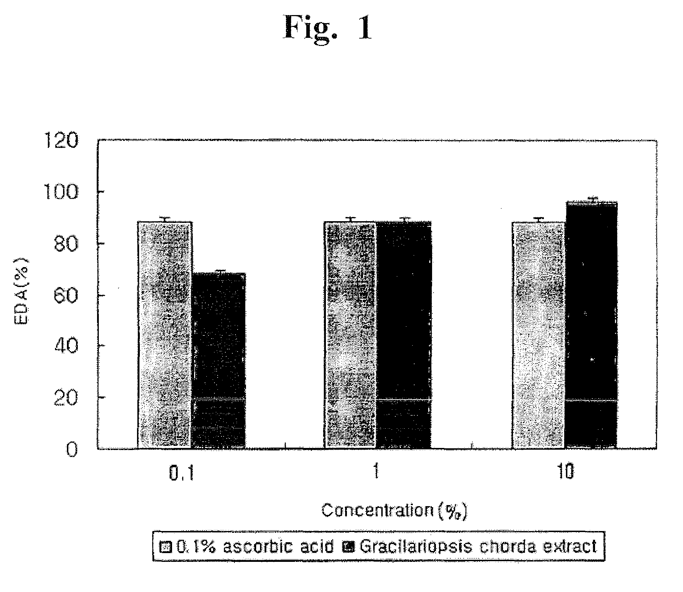

One ml of 0.2 mM DPPH ethanol solution was added to 2 ml extract diluents of above-mentioned Table 2, respectively. After thoroughly mixing for 10 sec at room temperature, the mixture was incubated for 30 min at 37°C. The absorbance was measured at 517 nm using a UV/VIS spectrometer, and EDA was determined according to the Formula below, as shown in Table 3 and Fig. 1.

$$EDA\ (\%) = \frac{\text{Absorbance after DPPH reaction} - \text{Absorbance in sample}}{\text{Absorbance in DPPH}} \times 100$$

Table 3.

| Free radical-scavenging ability (%) | | | |
|---|---|---|---|
| Ascorbic acid | Example 1 | Example 2 | Example 3 |
| 88% | 68% | 88% | 96% |

As shown in Table 3 above, all *Gracilariopsis chorda* extracts in Examples 1-3 of this invention have more than 68% EDAs, representing much higher scavenging ability. Moreover, it could be appreciated that EDA also is enhanced depending on increasing treatment concentrations of extract. Interestingly, 10% *Gracilariopsis chorda* extract of Example 3 exerted much high EDA (96%), suggesting that the present extract has strikingly anti-oxidative effects. Since UV generates free radicals in a skin cell to facilitate oxidation of cell membrane and to induce photoaging, it could be supposed that the present *Gracilariopsis chorda* extract effectively inhibits photoaging caused by UV.

4. Effect of *Gracilariopsis chorda* extract on UV-A absorption

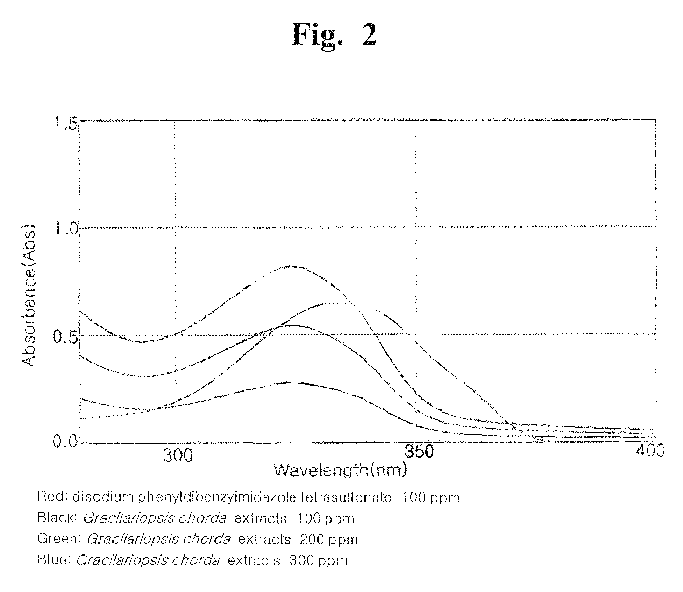

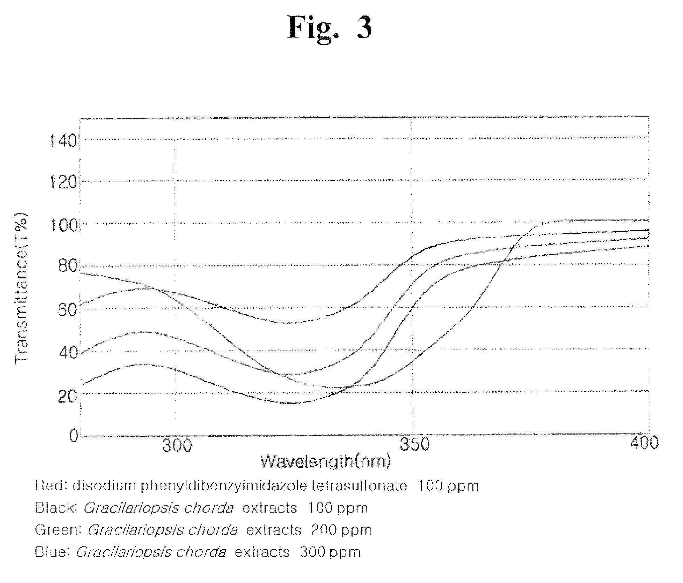

UV absorption capability was determined by measuring absorbance and transmission at a UV wavelength between 260 nm and 400 nm using a UV/VIS spectrometer. Practically, a chemical synthetic UV absorbent, disodium phenyldibenzyimidazole tetrasulfonate, was diluted with purified water at a concentration of 100 ppm, and also *Gracilariopsis chorda* extracts prepared in Preparation Example 1 at concentrations of 100 ppm, 200 ppm and 300 ppm. Considering purified water as a baseline, UV absorbance and transmittance spectra of the chemical synthetic UV absorbent and *Gracilariopsis chorda* extract were measured and shown in Fig. 2 and Fig. 3, respectively. In Figs. 2-3, maximal absorption of disodium phenyldibenzyimidazole tetrasulfonate and *Gracilariopsis chorda* extract was measured at 334 nm and 324 nm, respectively. In addition, 100 ppm chemical synthetic UV absorbent and 300 ppm *Gracilariopsis chorda* extract exhibited great UV absorption, suggesting that the natural extract of the present invention has much excellent activity. In view of UV transmission in wavelength between 300 nm and 350 nm, *Gracilariopsis chorda* extract inhibited at least 20% UV transmittance. Particularly, 300 ppm *Gracilariopsis chorda* extract prevented not less than 80% UV transmittance in a significant manner.

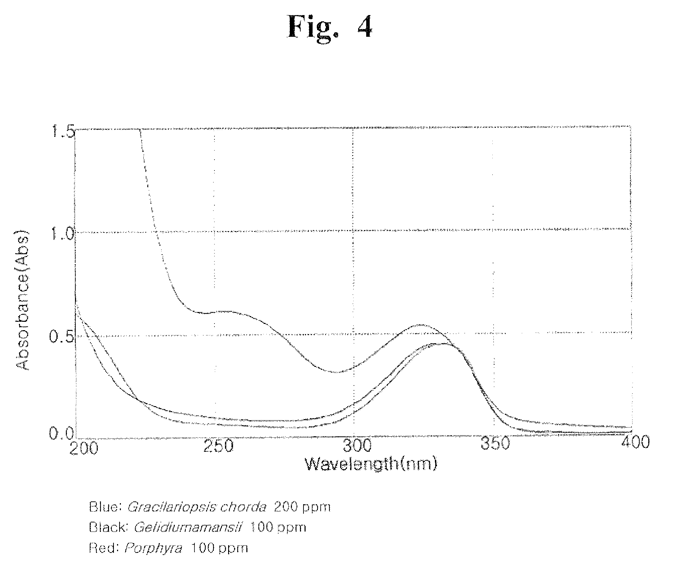

In addition to *Gracilariopsis chorda* extract, other red algae extracts, *Porphyra tenera* extract and *Gelidium Cartilagineum* extract prepared in Comparative Preparation Examples 1-2, were diluted at the same concentrations and their absorbing spectra were measured according to the aforementioned method. As results shown in Fig. 4, UV absorption of *Gracilariopsis chorda* extract at the same concentration was more excellent than that of other red algae extracts. Moreover, *Gracilariopsis chorda* extract exhibited much broad absorbing spectra in whole UV regions as well as certain wavelength, suggesting that *Gracilariopsis chorda* extract of this invention have more superior activity for blocking UV than other red algae extracts.

**<u>Preparation of a Lotion Containing _Gracilariopsis chorda_ Extract for Blocking UV, and UV-Blocking Effect on Skin Safety Test against Skin, and Stability Experiment of Cosmetic Powder Using the Same</u>**

**1. Preparation of a lotion containing _Gracilariopsis chorda_ extract for blocking UV**

As a component and composition described in Table 4 below, each UV-blocking lotion in Examples 4-6 contains _Gracilariopsis chorda_ extract prepared in Preparation Example 1 with a concentration of 5.0, 10.0, 15.0% by weight. In addition, a conventional UV-blocking lotion without _Gracilariopsis chorda_ extract (Comparative Example 1.) and _Porphyra tenera_ extract and _Gelidium Cartilagineum_ extract prepared in Comparative Preparation Examples 1-2 (Comparative Example 2 and Comparative Example 3, respectively) were prepared.

Practical preparation methods are as follows: according to conventional methods, components 1-16 and 20 were added in order and solubilized through heating by 70°C. After emulsification, the solution was cooled at room temperature and _Gracilariopsis chorda_ extract of component 17 was added and then prepared with agitation. Comparative Examples 2-3 were prepared according to the same method by adding component 18 or component 19 instead of Preparation Example 1.

**Table 4.**

| No. | component | Comparative Example 1 | Comparative Example 2 | Comparative Example 3 | Example 4 | Example 5 | Example 6 |
|---|---|---|---|---|---|---|---|
| 1 | octhyl methoxycinnamate | 6.0 | 6.0 | 6.0 | 6.0 | 6.0 | 6.0 |
| 2 | isoamyl p-methoxycinnamate | 2.0 | 2.0 | 2.0 | 2.0 | 2.0 | 2.0 |
| 3 | ozokerite (paraffin wax) | 1.0 | 1.0 | 1.0 | 1.0 | 1.0 | 1.0 |
| 4 | lauryl PEG-9 polydimethyl siloxyethyl dimethicone | 2.5 | 2.5 | 2.5 | 2.5 | 2.5 | 2.5 |
| 5 | PEG-10 dimethicone | 1.5 | 1.5 | 1.5 | 1.5 | 1.5 | 1.5 |
| 6 | Dicaprylyl carbonate | 7.0 | 7.0 | 7.0 | 7.0 | 7.0 | 7.0 |
| 7 | Caprylic/capric triglyceride | 8.0 | 8.0 | 8.0 | 8.0 | 8.0 | 8.0 |
| 8 | Propyl paraben | 0.1 | 0.1 | 0.1 | 0.1 | 0.1 | 0.1 |
| 9 | Methylene bis-benzotriazolyl tetramethyl butylphenol | 0.3 | 0.3 | 0.3 | 0.3 | 0.3 | 0.3 |
| 10 | Cyclopentasiloxane | 12.0 | 12.0 | 12.0 | 12.0 | 12.0 | 12.0 |
| 11 | Sequestering agents | 0.02 | 0.02 | 0.02 | 0.02 | 0.02 | 0.02 |
| 12 | Sodium chloride | 1.0 | 1.0 | 1.0 | 1.0 | 1.0 | 1.0 |
| 13 | 1,3-butylene glycol | 2.0 | 2.0 | 2.0 | 2.0 | 2.0 | 2.0 |
| 14 | Methyl paraben | 0.2 | 0.2 | 0.2 | 0.2 | 0.2 | 0.2 |
| 15 | Shiso extract | 0.5 | 0.5 | 0.5 | 0.5 | 0.5 | 0.5 |
| 16 | Phenoxyethanol | 0.5 | 0.5 | 0.5 | 0.5 | 0.5 | 0.5 |
| 17 | _Gracilariopsis chorda_ extract | - | - | - | 5.0 | 10.0 | 15.0 |
| 18 | _Porphyra tenera_ extract | - | 10.0 | - | - | - | - |

(continued)

| No. | component | Comparative Example 1 | Comparative Example 2 | Comparative Example 3 | Example 4 | Example 5 | Example 6 |
|---|---|---|---|---|---|---|---|
| 19 | *Gelidium Cartilagineum* extract | - | - | 10.0 | - | - | - |
| 20 | Purified water | Residual amount | Residual amount | Residual amount | Residual amount | Residual amount | Residual amount |
| (unit: % by weight) | | | | | | | |

## 2. Effect of UV Blocking on Skin

UV-blocking lotion of Examples 4-6 in Table 4 and Comparative Example 1 was applied to human body for assessing protection factor of UV-A (PEA). Testing method was carried out according to method and standard for measuring UV-blocking efficacy based on Article 9 of Cosmetics Law and Article 6 of Enforcement Regulation on Cosmetics.

Minimal persistent pigment darkening dose (MPPD) is determined according to standard described in Table 5 below, thereby calculating PEA. The calculation method utilizes Formula III described below. In the meantime, protection grade of UV-A (PA) is classified as Table 6 using the calculated PEA. These results were shown in Table 7.

$$PEA = \frac{MPPD \text{ of a region applied to sample (MPPDp)}}{MPPD \text{ of a region not applied to sample (MPPDu)}} \times 100$$

**Table 5.**

| Assessment | Description | Reference |
|---|---|---|
| 0 | No response | |
| ± | Case exhibiting slight response difficult to assess MPPD or not more than 50% region of an area illuminated with UV-A | |
| + | Case exhibiting MPPD in not less than 50% region of an area illuminated with UV-A compared with a region without UV-A illumination | MPPD |
| ++ | Case exhibiting 100% darkening response in an area illuminated with UV-A and a phenomena skin surface is swollen | |
| +++ | Case exhibiting 100% darkening response in an area illuminated with UV-A and a phenomena blister are generatd on the skin surface | |

**Table 6.**

| PFA | PA | UV-A blocking |
|---|---|---|
| ≥2, <4 | PA+ | positive |
| ≥4, < 8 | PA++ | More positive |
| ≥8 | PA+++ | Much more positive |

**Table 7.**

| Test sample | Testing Result | | |
|---|---|---|---|
| | PEA | PA | UV-A blocking |
| Comparative Example 1 | 3 | PA+ | Positive |
| Example 4 | 4 | PA++ | More positive |
| Example 5 | 5 | PA++ | More positive |
| Example 6 | 5 | PA++ | More positive |

As demonstrated in Table 7, it could be appreciated that Comparative Example 1 without *Gracilariopsis chorda* extract exhibits UV protection efficacies though it has low UV-blocking effects (PA+), whereas Examples 4-6 containing *Gracilariopsis chorda* extract of this invention significantly enhance UV-A blocking effects (PA++). Meanwhile, it may be supposed that difference on effects of *Gracilariopsis chorda* extract is not great depending on its content.

**3. Safety Test against Skin**

In first stimulatory skin test, *Gracilariopsis chorda* extract of Preparation Example 1, Comparative Example 1 of disodium phenyldibenzyimidazole tetrasulfonate and Examples 4-6 of Table 4 were patched with a dose (about 0.2 ml) to healthy adult men and women (each 10 persons) in a pin chamber for patch test for 48 hrs. Thereafter, pin chamber is removed and skin change was observed in naked eye at 30 min post-removal. These results were shown in Table 9 according to standard of Table 8.

**Table 8.**

| Symbol | Grade | Standard |
|---|---|---|
| + | 1 | Slight stimuli, erythema and erythematous spot, fever |
| ++ | 2 | Mild stimuli, systemic erythema |
| +++ | 3 | Strong stimuli, erythema accompanied by edema |
| ++++ | 4 | Strong stimuli, erythema and blister accompanied by edema |
| • Modified by Frosch & Kligman, Cosmetic, Toiletry and Fragrance Association (CTFA) standards | | |

**Table 9.**

| No. | Test sample | Judgement after 48hr |
|---|---|---|
| 1 | Preparation Example 1 | - |
| 2 | disodium phenyldibenzyimidazole tetrasulfonate | ++ |
| 3 | Comparative Example 1 | - |
| 4 | Example 4 | - |
| 5 | Example 5 | - |
| 6 | Example 6 | - |

As shown in Table 9, *Gracilariopsis chorda* extract of the present invention had no stimulatory effects on skin and disodium phenyldibenzyimidazole tetrasulfonate as an aqueous UV-absorbing agent utilized currently in the cosmetic powder field showed moderate skin stimulation accompanied by itching.

**4. Stability Experiment of Cosmetic Powder**

Among cosmetic powder compositions in Table 4, Examples 4-6 containing *Gracilariopsis chorda* extract with 5-15% concentrations, Comparative Example 1, and Comparative Example 2 and Comparative Example 3 containing *Porphyra tenera* extract and *Gelidium Cartilagineum* extract, respectively, was investigated during 2 weeks for stability depending on temperature to assess storage stability as cosmetics. The result was represented as Table 10.

**Table 10.**

| Classification | | Example 4 | Example 5 | Example 6 | Comparative Example 2 | Comparative Example 3 | Comparative Example 1 |
|---|---|---|---|---|---|---|---|
| -5°C | 1 week, 1month, 3 months | ◎◎◎ | ◎◎◎ | ◎◎◎ | ◎◎○ | ◎◎○ | ◎◎◎ |
| 4°C | | ◎◎◎ | ◎◎◎ | ◎◎◎ | ◎◎○ | ◎◎○ | ◎◎◎ |
| Room temperature | | ◎◎○ | ◎○○ | ○○○ | ○○× | ○×× | ◎◎◎ |
| 45°C | | ○○○ | ○○× | ○×× | ××× | ××× | ◎○○ |
| ◎: very favorable, ○: favorable, ×: color change, smell change | | | | | | | |

In view of physical state stability of cosmetic powder depending on temperature, 5% extract of this invention in Example 4 had most excellent stability, and thus it is sufficient to use the present composition as a cosmetic powder. At the same concentration, color and smell change in Example 2 and Comparative Examples 4-5 containing *Gracilariopsis chorda* extract is much lower than that in Comparative Examples 2-3 containing *Porphyry tenera* extract and *Gelidium Cartilagineum* extract, suggesting that *Gracilariopsis chorda* extract may be much useful as a cosmetic powder compared with other red algae such as laver or agar-agar.

Having described a preferred embodiment of the present invention, it is to be understood that variants and modifications thereof falling within the spirit of the invention may become apparent to those skilled in this art, and the scope of this invention is to be determined by appended claims and their equivalents.

**Claims**

1. A cosmetic composition for blocking ultraviolet (UV) rays, comprising *Gracilariopsis chorda* extract.

2. The cosmetic composition according to claim 1, wherein the composition comprises the *Gracilariopsis chorda* extract in a range of 0.1-30% by weight (wt%).

3. The cosmetic composition according to claim 1, wherein the composition further comprises laver (*Porphyra tenera*) extract or *Gelidium Cartilagineum* extract.

4. The cosmetic composition according to claim 1, wherein an UV-absorbing wavelength region in the composition is a long-wave ultraviolet (UV-A) region.

5. The cosmetic composition according to claim 1, wherein the composition comprises a lotion, a cream, a milky lotion or a gel to block UV.

6. A method for blocking UV, comprising administering in a local manner to skin of a subject the composition of any one according to claims 1 to 5.

# Fig. 1

# Fig. 2

Red: disodium phenyldibenzyimidazole tetrasulfonate 100 ppm
Black: *Gracilariopsis chorda* extracts 100 ppm
Green: *Gracilariopsis chorda* extracts 200 ppm
Blue: *Gracilariopsis chorda* extracts 300 ppm

# Fig. 3

Red: disodium phenyldibenzyimidazole tetrasulfonate 100 ppm
Black: *Gracilariopsis chorda* extracts 100 ppm
Green: *Gracilariopsis chorda* extracts 200 ppm
Blue: *Gracilariopsis chorda* extracts 300 ppm

# Fig. 4

Blue: *Gracilariopsis chorda* 200 ppm
Black: *Gelidiumamansii* 100 ppm
Red: *Porphyra* 100 ppm

<div align="center">INTERNATIONAL SEARCH REPORT</div>

| International application No. |
| --- |
| **PCT/KR2010/006338** |

| A. CLASSIFICATION OF SUBJECT MATTER |
| --- |
| ***A61K 8/97(2006.01)i, A61Q 17/04(2006.01)i, A61Q 19/00(2006.01)i*** |
| According to International Patent Classification (IPC) or to both national classification and IPC |

| B. FIELDS SEARCHED |
| --- |
| Minimum documentation searched (classification system followed by classification symbols) A61K 8/97; A61K 9/14; A61K 31/19; A61Q 19/02; A61K 36/03; A61K 36/02; A61K 36/04; A61P 17/18 |
| Documentation searched other than minimum documentation to the extent that such documents are included in the fields searched Korean Utility models and applications for Utility models: IPC as above Japanese Utility models and applications for Utility models: IPC as above |
| Electronic data base consulted during the international search (name of data base and, where practicable, search terms used) eKOMPASS (KIPO internal) & Keywords: Gracilariopsis chorda, ultraviolet rays blocking |

| C. DOCUMENTS CONSIDERED TO BE RELEVANT | | |
| --- | --- | --- |
| Category* | Citation of document, with indication, where appropriate, of the relevant passages | Relevant to claim No. |
| A | US 2008/0226740 A1 (CHEN RONG-HUEI et al.) 18 September 2008 See abstract; paragraphs [0047] and [0048]; claims 1 - 3, 21 - 25. | 1-6 |
| A | KR 10-0820291 B1 (CHEJU NATIONAL UNIVERSITY INDUSTRY-ACADEMIC COOPERATION FOUNDATION) 07 April 2008 See abstract; claim 1. | 1-6 |
| A | KR 10-0793081 B1 (CHEJU NATIONAL UNIVERSITY INDUSTRY-ACADEMIC COOPERATION FOUNDATION) 10 January 2008 See abstract; claims 7 and 10. | 1-6 |
| A | KR 10-0428817 B1 (KIM, WON GYU) 28 April 2004 See abstract; claims 1 and 2. | 1-6 |
| A | KR 10-2010-0021266 A (PUKYONG NATIONAL UNIVERSITY INDUSTRY-UNIVERSITY COOPERATION FOUNDATION) 24 February 2010 See abstract; claims 2 - 4, 6 - 11. | 1-6 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 26 JULY 2011 (26.07.2011) | **27 JULY 2011 (27.07.2011)** |
| Name and mailing address of the ISA/KR Korean Intellectual Property Office Government Complex-Daejeon, 139 Seonsa-ro, Daejeon 302-701, Republic of Korea Facsimile No. 82-42-472-7140 | Authorized officer Telephone No. |

Form PCT/ISA/210 (second sheet) (July 2009)

INTERNATIONAL SEARCH REPORT
Information on patent family members

International application No.

**PCT/KR2010/006338**

| Patent document cited in search report | Publication date | Patent family member | Publication date |
|---|---|---|---|
| US 2008/0226740 A1 | 18.09.2008 | NONE | |
| KR 10-0820291 B1 | 07.04.2008 | NONE | |
| KR 10-0793081 B1 | 10.01.2008 | NONE | |
| KR 10-0428817 B1 | 28.04.2004 | US 2003-0003063 A1 | 02.01.2003 |
| | | US 6485712 B1 | 26.11.2002 |
| KR 10-2010-0021266 A | 24.02.2010 | NONE | |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20100044593
- KR 1019950012443
- KR 1020060088347
- KR 1020060050782

**Non-patent literature cited in the description**

- **MOSMANN T et al.** Rapid Colorimetric Assay for Cellular Growth and Survival: Application to Proliferation and Cytotoxicity Assays. *J. Immunol. Meth.,* 1983, vol. 65, 55-63